# EUROPEAN PATENT SPECIFICATION

(11) **EP 2 739 301 B1**
(45) Date of publication and mention of the grant of the patent: **20.01.2016**
(21) Application number: 12745817.2
(22) Date of filing: 02.08.2012
(51) Int. Cl.: A61K 38/20, A61P 31/12, A61P 1/16

(54) **HCV IMMUNOTHERAPY**
HCV-IMMUNTHERAPIE
IMMUNOTHÉRAPIE CONTRE LE VHC

(30) Priority: 03.08.2011 EP 11306012; 04.08.2011 US 201161514915 P
(43) Date of publication of application: 11.06.2014
(73) Proprietor: Cytheris, 92130 Issy Les Moulineaux (FR)
(72) Inventor: MORRE, Michel, F-92100 Boulogne (FR); ASSOULINE, Brigitte, F-92400 Courbevoie (FR); CROUGHS, Thérèse, F-92130 Issy Les Moulineaux (FR); DEMOL, Pierre, F-78420 Carrieres Sur Seine (FR); BEQ, Stephanie, F-75012 Paris (FR)
(74) Representative: Chajmowicz, Marion
(86) International application number: PCT/EP2012/065125
(87) International publication number: WO 2013/017653

(56) References cited:
- MARC PELLEGRINI ET AL: "IL-7 Engages Multiple Mechanisms to Overcome Chronic Viral Infection and Limit Organ Pathology", CELL, vol. 144, no. 4, 1 February 2011 (2011-02-01), pages 601-613, XP009154942, ISSN: 0092-8674, DOI: 10.1016/j.cell.2011.01.011 cited in the application
- PARISH IAN A ET AL: "IL-7 knocks the socs off chronic viral infection.", CELL 18 FEB 2011 LNKD- PUBMED:21335230, vol. 144, no. 4, 18 February 2011 (2011-02-18), pages 467-468, XP002665877, ISSN: 1097-4172
- Cytheris: "IL-7 HCV Trials", , 2010, XP002665878, Retrieved from the Internet: URL:http://www.cytheris.com/Clinical_Trial s/hcv.php [retrieved on 2011-12-15]
- U.S. National Institutes of Health: "Dose Escalation Study of IL-7 and Bi-therapy in HCV Patients Resistant After 12 Weeks of Bi-therapy (ECLIPSE 1)", , 2 December 2009 (2009-12-02), XP002665879, Retrieved from the Internet: URL:http://clinicaltrials.gov/ct2/show/NCT 01025596?term=il-7+hcv&rank=3 [retrieved on 2011-12-15]
- U.S. National Institures of Health: "Dose Escalation Study of Interleukin-7 (IL-7) and Bitherapy in HCV Genotype 1 or 4 Patients Resistant to Bitherapy Alone (Eclipse 2)", , 2 December 2009 (2009-12-02), XP002665880, Retrieved from the Internet: URL:http://clinicaltrials.gov/ct2/show/NCT 01025297?term=il-7+hcv&rank=2 [retrieved on 2011-12-15]
- U.S. National Institutes of Health: "Dose Escalation Study of Interleukin-7 (IL-7) and Bitherapy in Asiatic HCV Patients Resistant to Bitherapy (ECLIPSE 3)", , 2 December 2009 (2009-12-02), XP002665881, Retrieved from the Internet: URL:http://clinicaltrials.gov/ct2/show/NCT 01024894?term=il-7+hcv&rank=1 [retrieved on 2011-12-15]
- Gill: "ECLIPSE 2 Hepatitis C Multicenter Study of IL-7 in Chronically Infected Genotype 1 and 4 Nonresponders", , 8 November 2011 (2011-11-08), XP002665882, Retrieved from the Internet: URL:http://hepcnet.net/boards/phorum/read. php?4,9671,9751 [retrieved on 2011-12-15]
- HABERSETZER FRANCOIS ET AL: "FOUR WEEKS OF IL-7 (CYT107) ADDED TO PEGINTERFERON (PEGIFN) AND RIBAVIRIN (RBV) IS SAFE, INDUCES A BROAD IMMUNE RESPONSE AND HCV VIRAL CLEARANCE IN GENOTYPE 1 AND 4 PATIENTS NON RESPONDERS TO PEGIFN AND RBV", HEPATOLOGY, vol. 54, no. Suppl. 1, October 2011 (2011-10), page 1432A, XP009154948, & 62ND ANNUAL MEETING OF THE AMERICAN-ASSOCIATION-FOR-THE-STUDY-OF-LIVE R-DISEASES (AASLD); SAN FRANCISCO, CA, USA; NOVEMBER 04 -08, 2011

## Description

The present invention relates to the field of hepatitis C treatment. More particularly it provides a new therapy against hepatitis C, using interleukin-7 (IL-7).

### Background of the invention:

Hepatitis C is the major cause of chronic liver disease and its complications including liver fibrosis and cirrhosis, liver failure and hepatocellular carcinoma.

Hepatitis C virus (HCV) is a major public health problem worldwide. The World Health Organization (WHO) estimates that up to 170 million individuals worldwide (3% of the world population) are infected with hepatitis C virus (HCV), more than 130 million of those individuals are chronically infected and at risk of developing liver cirrhosis and liver cancer. Around four million people become infected with HCV each year (World Health Organization. Viral cancers: Hepatitis C. online www.who.int; WHO 2010).

Today's standard-of-care (SOC) for eradication of HCV from the liver consist of Pegylated type I interferon (PegIFN) and synthetic nucleoside ribavirin (RBV) therapy (Fried MW et al; N Engl J Med. 2002; 347(13):975-82; EASL Clinical Practice Guideline: Management of hepatitis C virus infection, J Hepatol. 2011; 55:245-264). However, this standard therapy has limited and unpredictable efficacy, an extensive toxicity profile frequently leading to treatment discontinuation and is very expensive. Less than half of the chronically HCV-infected individuals of genotype 1 and 4 respond to long-term treatment (48 weeks) of standard therapy (PegIFN/RBV) (Testino G et al; Hepatogastroenterology 2011; 58(106):536-8).

Interferon (IFN) is a very active antiviral cytokine but it is lymphopenic, with a poor clinical tolerance. So while IFN exhibits antiviral activity, it also blocks the production and maintenance of long term protective central memory T cells. This translates to a high frequency of relapses in chronic HCV-infected patients treated with PegIFN/RBV. In addition, compared to a control group, extended treatment with peglnterferon in patients with advanced chronic hepatitis C is associated with excess overall mortality (Di Bisceglie AM et al; Hepatology 2011; 53(4):1100-8).

New antiviral compounds have been developed that target inhibition of different steps of the HCV life cycle. Several new antiviral drugs (small molecule inhibitors of viral replication also referred to as direct-acting antivirals (DAAs), including protease inhibitors and polymerase inhibitors) for hepatitis C, are currently in an advanced stages of development. Telaprevir and Boceprevir have reached the market (Ghany et al, Hepathology, 2011, 54(4):1433-1444). These new antiviral agents have been tested in monotherapy or in multidrug therapy, with or without standard of care (PegIFN/RBV). However, direct-acting antiviral monotherapy generally results in development of drug resistance which considerably reduces its effectiveness and leads to treatment failure. Drug resistance is a significant limitation to the use of DAAs. For example, Telaprevir (an NS3/4 protease inhibitor) monotherapy induces a viral load decrease of close to 99% within two days of therapy initiation but frequently, even though treatment continuation, there is a rebound in viral load within ten days (Kieffer TL et al; Hepatology; 2007 Sep; 46(3):631-9) due to emergence of drug resistance (Rong L et al; Sci Transl Med; 2010 May 5; 2(30):30ra32). Chronic infection is maintained by an elevated rate of mutation and a rapid turn-over of hepatitis C viruses, mostly in the liver. This high variability and diversity of the hepatitis C virus causes resistance to one or multiple classes of DAAs. Consequently, most treatments fail because of replication of variants resistant to antiviral agents. On the other hand, direct-acting antiviral drugs in mono- or combination therapy have shown their potential to increase the response rate and/or shorten the treatment duration, but they only work as an add on therapy together with PegIFN/RBV (McHutchison JG et al; N Engl J Med. 2009; 360(18):1827-38). The efficacy of these combined therapies has been demonstrated only for genotype-1 infection. Furthermore, they induce more side effects and increase the cost of treatment. Finally, their efficacy remains uncertain in terms of potential drug resistance issues.

Several immune-modulating agents (among which are monoclonal antibodies, cytokines such as the new interferon lambda, vaccines, and TLR agonists) capable of stimulating a general and specific immune response against HCV are also in development.

A number of scientific groups are currently working to develop both T cell and antibody based vaccines to prevent and also to treat HCV infection, but no vaccine exists so far. Furthermore, it may not be possible to develop a vaccine that targets all HCV genotypes because of the high degree of genetic diversity exhibited by the virus.

IL-7, a cytokine that is critical for T cell development and homeostasis, has exhibited interesting antiviral activity in preclinical models of chronic LCMV (Mice infected with LCMV clone-13 having persistent high-level viremia), but this activity only develops at very high doses of IL-7 which are not appropriate for testing in patients (Pellegrini M et al; Cell 2011; 144(4):601-13).

Despite the fact that the various therapies to control the virus have been improved over the past decade, limitations still remain, among which are treatment duration; treatment efficacy in curing chronic HCV; treatment tolerability, excessive cost and inadequate access. Not all HCV-infected patients benefit from antiviral treatment. None of the treatments proposed so far are able to offer a broad response rate over a very short term (weeks) - along with a prolonged effect providing protection from relapses. Today, non-responder HCV-infected patients have limited treatment options. An improved therapy is thus needed to treat HCV infection and HCV-related diseases and deaths.

### Summary of the invention:

The invention proposes IL-7 immunotherapy to stimulate an efficient immune response against a HCV virus, in combination with a short antiviral treatment that decreases the circulating HCV virus concentration.

The invention provides IL-7, for use in treating hepatitis C in a patient infected with hepatitis C virus, in combination or subsequently, with an antiviral agent or a combination of antiviral agents.

The antiviral agent or combination of antiviral agents is in a therapeutically effective amount that reduces HCV viral load to less than 5 Log₁₀ IU/mL, preferably less than 4 Log₁₀ IU/mL, more preferably less than 3 Log₁₀ IU/mL. IL-7 is used in a patient who has been treated with an antiviral agent or a combination of antiviral agents, so as to reduce viral load, before administration with IL-7.

It is thus provided a new therapeutic regimen for treating or inhibiting Hepatitis C infection in a human subject in need thereof, comprising:
- administering an antiviral treatment to decrease hepatitis C viral load and
- administering Interleukin-7 pharmaceutical composition to restore immune functions and provide a durable cure after discontinuation of therapy.

The antiviral agent or combination of antiviral agents reduces the viral load to less than 5 Log₁₀ IU/mL, preferably less than 4 Log₁₀ IU/mL, more preferably less than 3 Log₁₀ IU/mL, before administration with IL-7.

The antiviral agent is advantageously selected from the group consisting of an interferon, a protease inhibitor, a polymerase inhibitor, an inhibitor of virus entry, a helicase inhibitor, and ribavirin, or combinations thereof.

In other words, the invention relates to the use of Interleukin-7 (IL-7), for the manufacture of a medicament for treating hepatitis C in a patient infected with hepatitis C virus, in combination or subsequently, with an antiviral agent or a combination of antiviral agents.

It is described a method for treating hepatitis C in a patient infected with hepatitis C virus (HCV), which method comprises administering the patient with a therapeutically effective amount of an antiviral agent or of a combination of antiviral agents so as to reduce HCV viral load, while administering the patient with a therapeutically effective amount of IL-7 so as to stimulate an efficient immune response against the residual virus.

The treatment with the antiviral agent or the combination of antiviral agents is started before the treatment with IL-7, and preferably maintained during at least part of the treatment with IL-7, preferably during 6 to 12 weeks.

The invention makes it possible to induce a broad and stable antiviral immune response targeting many viral quasi species, blocking viral escape by mutation, and preventing HCV relapse after treatment completion or discontinuation.

The invention allows to broaden the repertoire of the specific immune response in the patients (i.e. the diversity of the TCR repertoire is broadened). This results in preventing relapses.

A rapid and effective antiviral response develops, and viral clearance is obtained.

In addition, sustained protection is achieved and supported by production of long term central memory T cells specific to the host (patient) virus.

### Legends to the Figures:

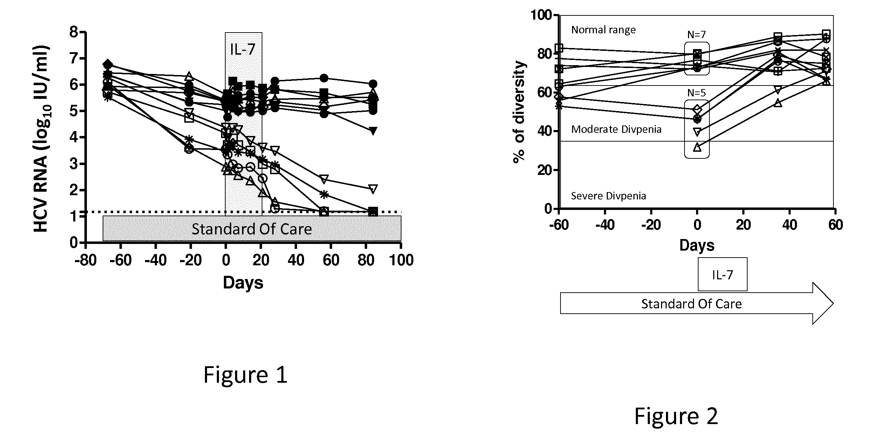

**Figure 1** is a graph showing the evolution of HCV viral load as determined by quantification of HCV RNA over time (days) in 12 patients subjected to 52 weeks of standard pegIFN+RBV (ribavirin) therapy (initiated 9 weeks (median) before IL-7 therapy to confirm lack of response to standard therapy), to which a short cycle of IL-7 (CYT107) was added (10µg/kg, once a week, for 4 weeks starting at Day 0).
   Patients who cleared the HCV virus decreased their HCV viral load by 2 Log₁₀ IU/mL (mean) between screening and D0 and had a viral load lower than 5 Log₁₀ IU/mL before IL-7 therapy.
**Figure 2** is a graph showing the evolution of T cell diversity in 12 patients subjected to 52 weeks standard pegIFN+RBV (ribavirin) therapy (initiated 9 weeks (median) before IL-7 therapy to confirm lack of response to standard therapy), to which a short cycle of IL-7 (CYT107) was added (10µg/kg, once a week, for 4 weeks starting at Day 0).
   5/12 patients were divpenic, implying that they exhibited moderate to severe reduction of immune diversity, before IL-7 therapy. After IL-7 therapy, normal T cell diversity was restored in all patients and remained stable at least until D56.

### Detailed description of the invention:

It is herein described a method for treating hepatitis C in a patient infected with a HCV virus, which method comprises administering interleukin-7 (IL-7) as an add-on therapy in said patient.

Surprisingly, by testing various associations in various patient populations, the inventors have found that while IL-7 therapy seems inactive in chronic HCV infection and unable to clear the virus in patients with commonly observed high viral loads (i.e. HCV RNA greater than 5 Log₁₀ IU/mL, generaly between 5 to 7 Log₁₀ IU/mL), if an antiviral agent is used to decrease the viral load to moderate or low levels (i.e. HCV RNA lower than 5 Log₁₀ IU/mL, preferably lower than 4 Log₁₀ IU/mL) then a short additive IL-7 therapy can (1) develop an interesting antiviral activity and quickly clear the virus in most patients, (2) enlarge T cell count, diversity and functionality, and, (3) induce an efficient and stable immune response, avoiding HCV relapse after treatment discontinuation or completion. The additive IL-7 therapy can also prevent liver hepatitis C-associated fibrosis and minimize risk of cirrhosis.

This was well demonstrated in chronic HCV patients, previously identified as non-responders to standard therapy (PegIFN/RBV), who showed a moderate decrease in their viral load after re-introduction of standard therapy and cleared the virus with the addition of IL-7 therapy when the viral load dropped below 4 Log₁₀ IU/mL.

Hepatitis C is a viral hepatitis resulting from an infection by a Hepatitis C virus (HCV). Any HCV strain or genotype (1, 2, 3, 4, 5, 6) is contemplated herein. Preferably the patient is infected with HCV genotype 1 or 4.

In the context of the invention, the term "treating" or "treatment", as used herein, means curing, reversing, alleviating, inhibiting the progress of, or preventing the disorder or condition to which such term applies, or one or more symptoms of such disorder or condition. The term "curing" preferably means that viral clearance is observed.
By "reducing viral load" is meant reducing the quantity of circulating HCV virus that can be measured, e.g. by quantitative RT-PCR. Viral load is expressed in Log₁₀ IU/mL. According to the invention, the term "patient" or "patient in need thereof" is intended for a human or non-human mammal infected or likely to be infected with HCV. The patient may be a male or a female, of any age, including children or teenagers. The patient may be asymptomatic, or may show early or advanced signs of hepatitis. In a particular embodiment, the patient shows a high HCV viral load when he begins treatment with the antiviral agent. A "high HCV viral load" means generally greater than 2 Log₁₀ IU/mL, still preferably greater than 3 Log₁₀ IU/mL, more preferably greater than 4 Log₁₀ IU/mL, still more preferably greater than 5 Log₁₀ IU/mL
In another embodiment, any patient, regardless of his/her HCV viral load, may benefit from the treatment of the invention.

### Antiviral agents:

The HCV viral load is reduced to below about 5 Log₁₀ IU/mL, preferably to below about 4 Log₁₀ IU/mL, more preferably to below about 3 Log₁₀ IU/mL during a first phase of treatment with an antiviral agent or a combination of antiviral agents.

In a particular embodiment, the antiviral agent may include interferon, ribavirin, inhibitors of the HCV protease, inhibitors of HCV polymerase (including nucleoside, nucleotide and non-nucleoside polymerase inhibitors), HCV virus entry inhibitors, helicase inhibitors and a combination thereof. Interferon (IFN) includes, but is not limited to, pegylated or not: IFN alpha comprising an IFN alpha variant such as IFN alpha-2a or IFN alpha-2b, IFN lambda or IFN omega, especially interferon alpha-2a, and even preferably pegylated Interferon alpha-2a, combined or not with ribavirin. Pegylated Interferon alpha-2a combined with ribavirin is currently the standard treatment. Combinations of interferon, associated or not with ribavirin, with inhibitors of the HCV protease or inhibitors of HCV polymerase, are also contemplated. Alternatively, combinations of direct-acting antivirals (DAAs), preferably at least one inhibitor of the HCV protease and at least one inhibitor of HCV polymerase, may be used as antiviral agents.

Generally speaking, the antiviral treatment may comprise any of the below mentioned drugs, especially interferon, ribavirin, inhibitors of the HCV protease, inhibitors of HCV polymerase (including nucleoside, nucleotide and non-nucleoside polymerase inhibitors), entry inhibitors, helicase inhibitors, and other anti-hepatitis C agents, or combinations thereof: (1) Interferon and/or ribavirin; (2) Substrate-based NS3 protease inhibitors (WO 98/22496); (3) Non-substrate-based inhibitors such as 2,4,6-trihydroxy-3-nitro-benzamide derivatives (Sudo K. et al., Biochemical and Biophysical Research Communications, 238:643-647 (1997); Sudo K., et al. Antiviral Chemistry and Chemotherapy, 9:186 (1998)), including RD3-4082 and RD3-4078, the former substituted on the amide with a 14 carbon chain and the latter processing a para-phenoxyphenyl group; (4) Thiazolidine derivatives, which show relevant inhibition in a reverse-phase HPLC assay with an NS3/4A fusion protein and NS5A/5B substrate (Sudo K. et al., Antiviral Research, 32: 9-18 (1996)), especially compound RD-1-6250, possessing a fused cinnamoyl moiety substituted with a long alkyl chain, RD4 6205 and RD4 6193; (5) Thiazolidines and benzanilides, identified in Kakiuchi N. et al. J. FEBS Letters 421, 217-220; and Takeshita N. et al. Analytical Biochemistry, 247: 242-246 (1997); (6) A phenanthrenequinone, which possesses activity against protease in a SDS-PAGE and autoradiography assay and is isolated from the fermentation culture broth of Streptomyces sp., Sch 68631 (Chu M. et al., Tetrahedron Letters, 37: 7229-7232 (1996)), and Sch 351633, isolated from the fungus Penicillium griscofuluum, which demonstrates activity in a scintillation proximity assay; (7) Selective NS3 inhibitors based on the macromolecule elgin c, isolated from leech (Qasim M. A. et al., Biochemistry, 36: 1598-1607 (1997)); (8) Helicase inhibitors (U.S. Pat. No. 5,633,358); (9) Polymerase inhibitors, such as nucleotide analogues, gliotoxin (Ferrari E. et al., Journal of Virology, 73:1649-1654 (1999)), and the natural product cerulenin (Lohmann V. et al., Virology, 249: 108-118 (1998)); (10) Antisense phosphorothioate oligodeoxynucleotides (S-ODN) complementary to sequence stretches in the 5' noncoding region (NCR) of the virus, or nucleotides 326-348 comprising the 3' end of the NCR and nucleotides 371-388 located in the core coding region of the HCV RNA; (11) Inhibitors of IRES-dependent translation; (12) Nuclease-resistant ribozymes; and (13) Miscellaneous compounds including 1-amino-alkyloyclohexanes (U.S. Pat. No. 6,034,134 to Gold et al.), alkyl lipids (U.S. Pat. No. 5,922,757 to Chojkier et al.), vitamin E and other antioxidants (U.S. Pat. No. 5,922,757 to Chojkier et al.), squalene, amantadine, bile acids (U.S. Pat. No. 5,846,964 to Ozeki et al.), N-(phosphonoacetyl)-L-aspartic acid, (U.S. Pat. No. 5,830,905 to Diana et al.), benzenedicarboxamides (U.S. Pat. No. 5,633,388 to Diana et al.), polyadenylic acid derivatives (U.S. Pat. No. 5,496,546 to Wang et al.), 2',3' dideoxyinosine (U.S. Pat. No. 5,026,687 to Yarchoan et al.), and benzimidazoles (U.S. Pat. No. 5,891,874 to Colacino et al.).

More recently, other anti-viral drugs, also named direct-acting antivirals (DAAs), have been developed, mainly depending on polymerase and protease enzymes as targets, and which may be used as antiviral agents as well:
(1) Protease inhibitors such as telaprevir (VX-950) which is a specific peptidomimetic inhibitor of NS3/NS4a protease (Reesink HW Gastroenterology 2006, 131:997-1002) and boceprevir (SCHS03034) (Sarrazin C Gastroenterology 2007, 132:1270-1278). Other protease inhibitors of interest include danoprevir, vaniprevir.
(2) Polymerase inhibitors of 2' and 3' substituted ribonucleoside analogues such as Valopicitabine, a prodrug of the nucleoside analogue 2-C-methylcytidine (NM283) (Pierra C J med chem. 2006, 49:6614-6620), and non nucleoside RNA-dependent RNA polymerase inhibitors, such as benzimidazole derivatives JTK-109 and JTK-003 (Tomei L. J Virology 2004, 78(2):938-946).

Non-nucleoside polymerase inhibitors include tegobuvir, filibuvir.

Nucleoside or nucleotide polymerase inhibitors include RG7128, PSI-7977.

Immune modulators capable of inducing an anti-viral response have been developed as well, including the Toll-like receptor agonists such as isatoribine (TLR7) (Horsmans Y, Hepatology 2005, 42:724-731), resiquimod (TLR7 and 8) (Pockros PJ, Hepatology 2007, 47:174-182), and CPG10101 (TLR9) (McHutchison JG, Hepatology 2007, 46:1341-1349).

The antiviral agent preferably is a direct-acting antiviral (DAA) or interferon or ribavirin, used either alone, together or in combination with other antiviral agents. Telaprevir and boceprevir are preferred protease inhibitors useful in the present invention.

Preferred combinations include (i) interferon and ribavirin, (ii) interferon, ribavirin and DAA(s), (iii) interferon and DAA(s), (iv) ribavirin and DAA(s).

Interferons (IFNs) are a well known family of cytokines secreted by a large variety of eukaryotic cells upon exposure to various mitogens. The interferons have been classified by their chemical and biological characteristics into four groups: IFN-alpha (leukocytes), IFN-beta (fibroblasts), IFN-gamma (lymphocytes), and IFN-lambda. IFN-alpha and beta are known as Type I interferons; IFN-gamma is known as Type II or immune interferon and IFN-Lambda is known as Type III interferon. Type I IFNs and Type III IFNs exhibit strikingly similar biological activities. Type III IFNs (lambda interferon (IFN-λ) or interleukin-28/29), display IFN-like activities, although they exert their action through a receptor complex distinct from the type I IFNs. The IFNs exhibit anti-viral, immunoregulatory, and antiproliferative activities. In the present invention, the interferon to use preferably is interferon-alpha.

Typical suitable interferon-alphas include, but are not limited to, recombinant IFN α-2b such as INTRON A interferon available from Schering Corporation, Kenilworth, N.J., recombinant IFNα-2a such as ROFERON® interferon available from Hoffmann-La Roche, Nutley, N.J., recombinant IFN-α 2c such as Berofor® alpha 2 interferon available from Boehringer Ingelheim Pharmaceutical, Inc., Ridgefield, Conn. IFN-α n1, a purified blend of natural alfa interferons such as SUMIFERON® available from Sumitomo, Japan or as WELLFERON® IFN-α n1 (INS) available from the Glaxo-Wellcome Ltd., London, Great Britain, or a consensus alpha interferon such as those described in U.S. Pat. Nos. 4,897,471 and 4,695,623 (especially Examples 7, 8 or 9 thereof) and the specific product available from Amgen, Inc., Newbury Park, Calif., or IFN-α n3, a mixture of natural alfa interferons made by Interferon Sciences and available from the Purdue Frederick Co., Norwalk, Conn., as ALFERON® or recombinant interferon alpha available from Frauenhoffer Institute, Germany or that is available from Green Cross, South Korea. Using IFN α -2b or IFN α -2a is preferred.In a most preferred embodiment, the interferon is in PEGylated form. A PEGylated interferon is a polyethylene glycol modified conjugate of interferon.

Polyethylene-glycol-interferon alfa-2a conjugate is preferred (see EP 809 996), such as PEGASYS®.

PEGylated interferon lambda may also be used (as developed by Bristol Myers Squibb for instance).

Furthermore, interferon may be fused or conjugated to a protein such as albumin. For instance, albumin interferon alfa-b (alb-IFN) (Albuferon®) is a polypeptide molecule that combines the therapeutic activity of interferon alpha with the long half-life of human serum albumin.

In still a preferred embodiment, interferon is used no more than six weeks, especially interferon is used no more than three weeks after the IL-7 treatment.

Indeed, in the present invention, the antiviral agent is preferably a direct-acting antiviral (DAA) agent targeting the HCV viral genotype of the patient such as a protease inhibitor or a polymerase inhibitor, and preferably a combination thereof.

### Interleukin 7:

Within the context of the present invention, "IL-7" designates a mammalian (e.g., human, simian, bovine, equine, feline or canine) IL-7 polypeptide. More preferably, the IL-7 polypeptide is a human IL-7 polypeptide.

Preferred human IL-7 polypeptides of this invention comprise an amino acid sequence as described in EP 314 415 or in WO2004/018681 A2, as well as any natural variants and homologs thereof. The sequence of human IL-7 is also available on gene banks. The typical wild-type protein comprises 152 amino acids and, optionally, an additional N-terminal methionine residue. Variants thereof include, more preferably, natural allelic variants resulting from natural polymorphism, including SNPs, splicing variants, etc.

The IL-7 polypeptide used in the present invention is preferably a recombinant IL-7. The term "recombinant", as used herein, means that the polypeptide is obtained or derived from a recombinant expression system, i.e., from a culture of host cells (e.g., microbial or insect or plant or mammalian) or from transgenic plants or animals engineered to contain a nucleic acid molecule encoding an IL-7 polypeptide. "Microbial" refers to recombinant proteins made in bacterial expression systems. "Mammalian" refers to recombinant glycoproteins made in mammalian expression systems. All of these host cells should preferably express either naturally or after transgenesis an appropriate glycosyltransferase and/or sialyltransferase gene. IL-7 polypeptide can also be glycosylated through the use of appropriate in vitro or in vivo glycosyltransferase and/or sialyltransferase molecules, or by grafting oligosaccharide structures. CHO cells are preferred.

A specific example of a human IL-7 polypeptide is a polypeptide of SEQ ID NO: 1 comprising the disulfide bridges Cys2-Cys92; Cys34-Cys129 and Cys47-Cys141, as described in EP 1 527 179.

Also, IL-7 polypeptides of the present invention may comprise the sequence of a mature IL-7 polypeptide, or further comprise additional amino acid residues, such as a secretion peptide for instance. Preferred examples of such secretion peptides include, without limitation, a signal peptide selected from the group consisting of the EPO signal peptide, SEAP signal peptide, IgGkappa signal peptide, Lactotransferin/vitronectin signal peptide, VIP/vitronectin signal peptide and cytostatin bis signal peptide.

In a preferred embodiment, IL-7 is in hyperglycosylated form, as described in WO2007/010401.

Within the context of the present invention, the term "hyperglycosylated IL-7" designates an IL-7 polypeptide having at least three glycosylated amino acid residues, an average isoelectric point inferior to 6.5 and an average molecular weight superior to 27 KDa as determined by SDS gel electrophoresis.

The structure and number of oligosaccharide units attached to a particular glycosylation site in the hyperglycosylated IL-7 polypeptide can be variable. These may be, for instance, N-acetyl glucosamine, N-acetyl galactosamine, mannose, galactose, glucose, fucose, xylose, glucuronic acid, iduronic acid and/or sialic acids.

More preferably, hyperglycosylated IL-7 polypeptides comprise N-linked and/or O-linked carbohydrate chain(s) selected from:
a) a mammalian type sugar chain, preferably of the type expressed by CHO cells;
b) a sugar chain comprising a complex N-carbohydrate chain (e.g., a triantenary or biantenary structure), more preferably containing high mannose and acetylglucosamine molecules and high terminal sialic acid residues;
c) a sugar chain comprising an O-carbohydrate chain without and preferably with a terminal sialic acid residue;
d) a sugar chain sialylated by alpha2,6-sialyltransferase or alpha2,3-sialyltransferase ; and/or
e) a sialylated sugar chain displaying between 3 to 30 sialyl-N-acetylgalactosamine, preferably 7 to 23.

Particularly preferred carbohydrate chain(s) comprise a triantenary or biantenary structure with partial or complete terminal sialylation. Further preferred carbohydrate chains comprise triantenary structures and tri or bi-sialylation, and/or a diantenary structure with disialylation.

The hyperglycosylated interleukin-7 polypeptide of interest advantageously has an average isoelectric point inferior to 6,5 and an average apparent molecular weight superior to 27 kDa, between 28 KDa and 65 KDa (theroretical for a 7N + 1O glycosylation), preferably between 28 KDa and 35 KDa (as shown for a 3N + 1O glycosylation), by gel electrophoresis (confirmed by Western blot) which is translated to 25 kDa by mass spectrometry analysis.

A "glycosylation site" designates any amino acid residue or region in a polypeptide which is subject to glycosylation, i.e., the attachment of a carbohydrate structure. Such sites are typically N-glycosylation sites (i.e., any amino acid residue or region in a polypeptide which allows the attachment of a carbohydrate structure through N-linkage) and/or O-glycosylation sites (i.e., any amino acid residue or region in a polypeptide which allows the attachment of a carbohydrate structure through O-linkage). Consensus sequences for glycosylation sites are known per se in the art. As an illustration, a consensus N-glycosylation site typically has the following structure: Asn-X-Ser/Thr, where X is any amino acid except Proline. Such glycosylation sites may be either naturally present within an IL-7 polypeptide sequence and/or artificially added or created within said sequence.

A preferred IL-7 composition useful in the present invention comprises at least 80 % human IL-7 recombinant polypeptides having at least three glycosylated amino acid residues, an average isoelectric point inferior to 6.5 and an average molecular weight superior to 27 KDa as determined by SDS gel electrophoresis, and comprising the disulfide bridges Cys2-Cys92; Cys34-Cys129 and Cys47-Cys141.

The IL-7 polypeptides preferably are N-glycosylated on at least three distinct amino acid residues.

In another preferred embodiment, IL-7 is fused to another protein entity. Examples of IL-7 fusion proteins are described in WO2005/063820. For instance it is in the form of an IL-7 fusion protein such as (1) an IL-7 functionally attached to a Fc portion of an IgG heavy chain, typically through a peptide hinge region, and the IgG moiety is preferably a human IgG1 or IgG4 as described in WO2007/010401, (2) a fusion protein as described in US patents.7,323,549 and 7,589,179, and US patent application 20090010875 , (3) an IL-7 functionally associated to a human serum albumin ("HSA") or a portion of a HSA, as a fusion protein, as described in WO2007/010401, or (4) an IL-7 functionnally associated to Human Growth Facteor (HGF) or a portion thereof, as a fusion protein.

IL-7 variants are encompassed, that show substantial amino acid sequence identity to wild-type mature mammalian IL-7s and substantially equivalent biological activity, e.g., in standard bioassays or assays of IL-7 receptor binding affinity. For example, IL-7 refers to an amino acid sequence of a recombinant or non-recombinant polypeptide having an amino acid sequence of: i) a native or naturally-occurring allelic variant of an IL-7 polypeptide, ii) a biologically active fragment of an IL-7 polypeptide, iii) a biologically active polypeptide analog of an IL-7 polypeptide, or iv) a biologically active variant of an IL-7 polypeptide.

A "variant" of an IL-7 protein is defined as an amino acid sequence that is altered by one or more amino acids. The variant can have "conservative" changes, wherein a substituted amino acid has similar structural or chemical properties, e.g., replacement of leucine with isoleucine. More rarely, a variant can have "nonconservative" changes, e.g., replacement of a glycine with a tryptophan. Similar minor variations can also include amino acid deletions or insertions, or both. Guidance in determining which and how many amino acid residues may be substituted, inserted or deleted without abolishing biological activity can be found using computer programs well known in the art, for example software for molecular modeling or for producing alignments. The variant IL-7 proteins included within the invention include IL-7 proteins that retain IL-7 activity. IL-7 polypeptides which also include additions, substitutions or deletions are also included within the invention as long as the proteins retain substantially equivalent biological IL-7 activity. For example, truncations of IL-7 which retain comparable biological activity as the full length fonn of the IL-7 protein are included within the invention. The activity of the IL-7 protein can be measured using in vitro cellular proliferation assays. The activity of IL-7 variants of the invention maintain biological activity of at least 30%, at least 40%, 50%, 60%, 70%, preferably at least 80%, 90% , 95% or even 99% as compared to wild type IL-7.

Variant IL-7 proteins also include polypeptides that have at least about 70%, 75%, 80% , 85%, 90%, 95% more sequence identity with wild- type IL-7. To determine the percent identity of two amino acid sequences or of two nucleic acids, the sequences are aligned for optimal comparison purposes (e.g., gaps can be introduced in the sequence of a first amino acid or nucleic acid sequence for optimal alignment with a second amino acid or nucleic acid sequence). The percent identity between the two sequences is a function of the number of identical positions shared by the sequences (i.e., percent homology = # of identical positions/total # of positions.timesx100). The determination of percent homology between two sequences can be accomplished using a mathematical algorithm. A preferred, non-limiting example of a mathematical algorithm utilized for the comparison of two sequences is the algorithm of Karlin and Altschul (1990) Proc. Natl. Acad. Sci. USA 87:2264-68, modified as in Karlin and Altschul (1993) Proc. Natl. Acad. Sci. USA 90:5873-77. Such an algorithm is incorporated into the NBLAST and XBLAST programs of Altschul, et al. (1990) J. Mol. Biol. 215:403-10. BLAST nucleotide searches can be performed with the NBLAST program, score=100, wordlength=12. BLAST protein searches can be performed with the XBLAST program, score=50, wordlength=3. To obtain gapped alignments for comparison purposes, Gapped BLAST can be utilized as described in Altschul et al., (1997) Nucleic Acids Research 25(17):3389-3402. When utilizing BLAST and Gapped BLAST programs, the default parameters of the respective programs (e.g., XBLAST and NBLAST) can be used.

### Regimen:

According to the invention, IL-7 is to be administered preferably once or twice a week, preferably during a period of two to six weeks, preferably four weeks, which defines an IL-7 treatment cycle. Such cycle can be repeated at least once.

In a preferred embodiment, IL-7 is administered once a week during four weeks.

In a preferred embodiment, the treatment with the antiviral agent or the combination of antiviral agents is maintained during at least part, of the treatment with IL-7, preferably the treatment with the antiviral agent or combination of antiviral agents is not interrupted. Most preferably, IL-7 is to be administered in combination with the antiviral agent or combination of antiviral agents. IL-7 can then be administered separately, simultaneously or sequentially with the antiviral agent or combination of antiviral agents.

In a particular embodiment, IL-7 is administered simultaneously with the antiviral agent or combination of antiviral agents.

In a preferred protocol, the patient is to be administered with IL-7 before the antiviral agent or the combination of antiviral agents, preferably one week before.

In another preferred protocol, the patient is to be administered with IL-7 from the initiation of therapy at the same time as the antiviral agent or the combination of antiviral agents, preferably starting between D0 and D10, most preferably starting between D3 and D7.

In another preferred protocol, the patient is to be administered with an antiviral agent or a combination of antiviral agents during a first phase, that is preferably of at least one week duration, so as to reduce the viral load, followed by a second phase of preferably 2 to 6 weeks of IL-7, preferably combined with an antiviral agent or a combination of antiviral agents.

The administration of IL-7 may be followed by a third phase lasting at least 1 to 3 weeks, or may be extended beyond 4 or 6 weeks or more of treatment with an antiviral agent or a combination of antiviral agents. Preferably this third phase lasts 1 to 9 weeks.

Altogether the patient is advantageously administered with the antiviral agent or combination of antiviral agents for a period of 6 to 12 weeks.

The antiviral agent or combination of antiviral agents is preferably the same during all treatment phases. However it can be changed if desired.

In a preferred embodiment, the protocol involves a preliminary but quick decrease of the patient viral load, followed by the addition of a short term IL-7 therapy, while the above antiviral treatments are maintained over this period and for a few weeks afterwards. When the treatments are stopped, the patient's immune system can efficiently and stably control itself the HCV virus.

The amount of antiviral agent such as interferon may be from 2 to 10 million IU per week on a weekly, twice or three times a week, or daily basis. In a preferred embodiment, the interferon-alpha administered is interferon-alpha-2b and the amount of interferon is administered 3 million IU twice or three times a week.

In a particular embodiment, the interferon-alpha administered is a pegylated interferon alpha-2b and the amount of interferon administered is from 0.5 to 2.0 micrograms/kg body weight, per week on a weekly, twice or three times a week, or daily basis. Alternatively, the interferon administered is a pegylated interferon alpha-2a and the amount of interferon administered is from 20 to 250 micrograms/kilogram body weight per week on a weekly, twice or three times a week, or daily basis.

Other antiviral agents such as ribavirin may be administered from about 400 to about 1600 mg per day, preferably about 600 to about 1200 mg/day or about 800 to about 1200 mg day and most preferably about 1000 to about 1200 mg/kg a day based on the patient's weight.

Other antiviral agents such as telaprevir may be administered from about 750 mg three times a day (preferably 7-9 hours apart).

Other antiviral agents such as boceprevir may be administered from about 800mg three times a day (7-9 hours apart).

Preferably, the effective amount of interleukin-7 to be administered is comprised between about 3 to 30 µg/kg, preferably between about 5 to 20 µg/kg, and is preferably about 10µg/kg body weight, more preferably 20µg/kg body weight. Preferably it is administered on a weekly basis, preferably for 2 to 6 weeks.

If desired, IL-7 can be administered twice a week.

In preferred embodiments, IL-7 can be administered once a week, during a cyclic period of two to four weeks. The cycle could be repeated at least once.

IL-7 and the antiviral agent may be administered simultaneously, either separately or within the same formulation. Preferably, they are administered simultaneously, and both therapies may be initiated at the same time or IL-7 may be initiated one week before antiviral agent. More preferably, they are administered separately, according to different schedules. The antiviral agent dose is preferably administered during the same period of time that the patient receives doses of IL-7.

### Pharmaceutical compositions:

The pharmaceutical compositions comprising IL-7 may be suitable for oral, rectal, or parenteral routes, more particularly by intravenous, subcutaneous, intradermal, intraarterial, intra-peritoneal or intra-muscular, as well as intranasal route. The parenteral route, especially subcutaneous, is preferred. For instance, the active ingredient is associated with a pharmaceutically acceptable carrier, excipient or diluent which may be selected from neutral to slightly acidic, isotonic, buffered saline, solutions or suspensions and more preferably from sucrose, trehalose, and amino acid. The pharmaceutically compatible carrier is preferably contained in an appropriate buffer to form an isotonic solution. An appropriate buffer has preferably a pH range comprised between 4.5 to 7.5, preferably 5.0 to 7.0, even more preferably of about 5.5 and is preferably an organic salt selected from a sodium citrate buffer or an ammonium acetate buffer. The pharmaceutical composition may be in the form of a suspension, solution, gel, powder, solid, etc. The composition is preferably a liquid form.

The composition may comprise stabilizing agents, such as sugar, amino acids, proteins, surfactants, etc. The composition may comprise any saline solution, including phosphates, chloride, etc.

A particular pharmaceutical composition according to the invention comprises, in addition to the active drug substance, a protein and/or a surfactant. This presence of a protein, or any other high molecular weight molecule of natural origin, reduces exposition of IL-7 to the host immune system and therefore avoids secondary effects. More preferably, the protein is non immunogenic in the subject, such as any protein of human origin. A most preferred example of protein is human serum albumin. The surfactant may be selected from known surfactants such as Polysorbate products, preferably Tween20® or Tween80®. A specific composition of this invention comprises human serum albumin (preferably 2 to 5 mg/ml) or polysorbate (Tween 20 or 80 (typically 0.005%)) or any other substance such as a tensioactive substance or amino acid (e.g., arginine,, glutamate, or a mixture of arginine and glutamate) or sugar (e.g., sucrose, trehalose, sorbitol), capable of preventing IL-7 immunogenicity due to protein aggregation and/or local persistence of the drug product at injection site after administration of the composition.

In a particular embodiment, the administration route is the oral route. In comparison to other polypeptide hormones, oral route is indeed acceptable for IL-7, especially in hyperglycosylated form, because of the exceptional stability of this protein. The compositions can then be in a solid form, such as a tablet or a powder or a capsule, or in a form of a liquid, such as a syrup or an emulsion, prepared in an appropriate pharmaceutically acceptable carrier. Preferably the carrier itself is stable in the gastrointestinal tract and in the circulatory system and exhibits an acceptable plasma half-life. Gastric acid-resistant capsules, such as gastric acid-resistant capsules containing a micro-emulsion or liposome formulation of IL-7 polypeptide, are advantageous.

Additional active ingredients, such as immuno-stimulating agents, preferably selected from a hematopoietic cell growth factor, a cytokine, an antigenic molecule (or antigen) and an adjuvant, may be used for combined, separate or sequential use.

### Therapeutic indication:

The invention allows a dramatic reduction in the HCV viral load.

Viral clearance and alleviation of the symptoms may be observed within 1 week to 6 months, preferably within 1 week to 3 months after treatment.

The invention makes it possible to inhibit the progress of the disease, and to obtain a substantially complete clearance of the virus. In other words HCV RNA becomes undetectable in the patient.

The invention is particularly useful for preventing or delaying any deleterious evolution resulting from the HCV infection, in particular any onset of liver fibrosis or cirrhosis or hepatocarcinoma.

The protocol of the invention is of particular interest in a patient who has not responded to a prior treatment. These patients include non-responder patients (also called partial responders or slow responders) or null-responder patients. In particular, non-responder patients (also called partial responders or slow responders) are patients for whom HCV RNA has decreased by 2 logs t week 12 but does not become undetectable by week 24, after initiation of a treatment, especially a prior treatment with interferon alone, or a combination of ribavirin and interferon, which is currently the standard treatment. These patients are unlikely to achieve SVR (sustained viral response) even when retreated with standard therapy. Null-responders are patients for whom HCV RNA has not decreased by at least 1 log (a factor of 10) after 4 weeks of treatment, or by 2 logs after 12 weeks of treatment. These patients are extremely unlikely to achieve SVR even when retreated with standard therapy.

Absence of viral response to previous treatments is defined as null-response or absence of early viral response (EVR), defined by a decrease of HCV RNA loads lower than 2 logs after 12 weeks as measured by a quantitative RT PCR test, compared to baseline levels measured by a similar technique. Or, absence of end of treatment response defined by detectable HCV RNA at the end of treatment.

The protocol of the invention may be also advantageous for treating a naïve patient, i.e. a patient who has never been treated for an HCV infection, more particularly a patient who has never been treated with ribavirin or any interferon.

Patients with hepatitis C who have been treated for the infection, especially with ribavirin or any interferon, may also be good candidates for the combination therapy of the invention.

These include patients with hepatitis C who have relapsed after initial response to previous treatments.

Patients who show viral break-through can also benefit from the treatment of the invention. A viral break-through occurs when a patient achieves a response under therapy (especially therapy with interferon) but then loses the response despite the continuous therapy.

Patients having acute or chronic hepatitis C infections are encompassed, including relapsers, non-responders and null-responders.

In a particular embodiment, the patient has been genotyped for single-nucleotide polymorphism in the IL28b gene locus that encodes encoding interferon-lambda-3 (see Thomson et al, Gastroenterology. 2010, 139(1):120-9, and international patent application WO2011/013019). A CC genotype at SNP rs12979860 is indicative of a patient responsive to a SOC treatment, especially pegylated interferon-alpha (PEG-IFN-alpha) plus ribavirin (RBV) treatment. A CT or TT genotype is indicative of a non-responder or null-responder. In a preferred embodiment, a patient with a CT or TT genotype at SNP rs12979860 can advantageously benefit from the treatment of the present invention.

The protocol is useful against the high variability and diversity of hepatitis C viruses, avoiding emergence of resistance to treatment, benefiting more patients, and providing a faster, efficient and more sustained response.

The protocol of the invention may further be useful in a patient co-infected with HCV and another virus, such as HIV, HBV, HPV, HSV, or CMV.

Especially this method may be useful to HIV/HCV co-infected patients who present with low CD4 T cell counts (<400 CD4/µL) among which some cannot be treated due to their very low CD4 T cell counts (<250 CD4/µL), which is not compatible with interferon treatment.

In this case the same treatment regimen may be applied after a preparation cycle of about 2 to 4 weeks of IL-7 or any other IL-7 agonist to restore adequate CD4 T cell counts before applying the protocol described herein.

The protocol of the invention could further be adapted to the HCV/HBV co-infected patients who present with a detectable viral load of HBV. In this case a preliminary reduction of the HBV viral load could be obtained by a 3 to 4 month pretreatment with a direct anti HBV antiviral, such as entecavir or tenofovir.

The figures and examples illustrate the invention without limiting its scope.

### EXAMPLES

### Example 1: Evaluation in hepatitis C Liver disease of IL-7 in a Phase I/IIa Study

### Methods:

A Phase I/IIa study was designed to evaluate the safety and individual benefits of weekly doses of Interleukin-7 in adult patients infected by Genotype 1 or 4 Virus of Hepatitis C and resistant to current "Standard-Of-Care" (SOC) with Peg-Interferon and Ribavirin after 12 weeks of this standard bi-therapy.

Absence of viral response to current Standard-Of-Care with pegylated interferon-alpha + ribavirin, identified as absence of early viral response (EVR), is defined as a decrease of HCV RNA loads lower than 2 logs, as measured by a quantitative PCR test after 12 weeks of standard therapy, compared to baseline levels measured by a similar technique. Or, absence of end of treatment response defined by detectable HCV RNA at the end of treatment (24 weeks or 48 weeks).

In this open-label, dose-escalating study, (3, 10 and 20 µg/kg/week) CYT107 (recombinant human glycosylated IL-7) was administered by subcutaneous route for 4 weeks (D0 to D21) as an add on to 52 weeks SOC therapy initiated 9 weeks (median) before CYT107 to confirm lack of response to SOC. 6 Patients were included at each dose level and 6 more if at least 2 Patients had a HCV RNA drop > 2 logs.

### Results:

There were no serious Adverse Events or clinically relevant abnormalities in biological parameters related to CYT107 treatment.

At D56, CYT107 (10µg/kg/wk) induced (median values):
- a T cell increase +341 CD4/µl(+168%) and +209 CD8/µl (+179%) more than correcting the initial pre-CYT107-SOC induced lymphopenia (-147/µL CD4).
- a broadening of TCR repertoire diversity (+25%) in the 4 patients with low diversity at D0 (45%).
- an increased number of CD3 expressing the α4/β7 receptors (+ 73%)

These increases in T cell counts, diversity and homing were associated with an accelerated rate of HCV viral decrease and clearance at week 12 in 5/12 patients. Afterwards, HCV RNA remained undetectable (median current follow up: 11 months). Responding patients had a moderate viral load (<4.52 log/mL) at CYT107 initiation. ,

As shown on the Figure 1, the 7 patients unable to decrease their viral load during Standard-of-Care reintroduction did not clear the virus with IL-7 add on therapy (10 µg/kg, once a week, for 4 weeks starting at day 0), while the 5 patients dropping their viral loads below 5 Log₁₀ IU/mL under Standard bi-therapy, cleared the virus with the same IL-7 treatment (given at day 0).

Figure 2 shows that, after IL-7 therapy, normal T cell diversity was restored in all patients and remained stable at least until D56.

### Conclusions:

In chronic HCV patients defined as non-responders to standard bi-therapy with PEGinterferon and ribavirin, IL-7 treatment was safe and expanded both CD4 and CD8 T cells, an effect known to provide an efficient and stable immune response. IL-7 also contributed to an increase of T cell homing in lymphoid organs, and normalization of the diversity of the TCR repertoire. This effect was systematically associated with viral clearance in patients dropping their viral loads below 5 Log₁₀ IU/mL under the standard bi-therapy.

## Claims

1. Interleukin-7 (IL-7), for use in treating hepatitis C in a patient infected with hepatitis C virus, wherein the patient has been treated with an antiviral agent or a combination of antiviral agents, so as to reduce viral load, before administration with IL-7, wherein the antiviral agent or combination of antiviral agents reduces the viral load to less than 5 Log₁₀ IU/mL.

2. The IL-7 for use in treating hepatitis C according to claim 1, wherein the antiviral agent or combination of antiviral agents reduces the viral load to less than 4 Log₁₀ IU/mL before administration with IL-7.

3. The IL-7 for use in treating hepatitis C according to claim 2, wherein the antiviral agent or combination of antiviral agents reduces the viral load to less than 3 Log₁₀ IU/mL before administration with IL-7.

4. The IL-7 for use in treating hepatitis C according to any of claims 1 to 3, wherein the antiviral agent is selected from the group consisting of a protease inhibitor such as Telaprevir or Boceprevir, a polymerase inhibitor, an inhibitor of virus entry, and a helicase inhibitor, or combinations thereof, optionally in combination with interferon and/or ribavirin.

5. The IL-7 for use in treating hepatitis C according to any of claims 1 to 3, wherein the antiviral agent is interferon, such interferon alpha or consensus interferon or interferon lambda, preferably IFNalpha-2a or IFNalpha-2b, either alone or in combination with another antiviral agent, such as ribavirin, the interferon being preferably in PEGylated form.

6. The IL-7 for use in treating hepatitis C according to any of claims 1 to 5, wherein the treatment with the antiviral agent or the combination of antiviral agents is started before the treatment with IL-7, and maintained during at least part of the treatment with IL-7, preferably during 6 to 12 weeks.

7. The IL-7 for use in treating hepatitis C according to any of claims 1 to 6, wherein IL-7 is to be administered once a week, preferably during a period of two to six weeks, preferably four weeks, thereby defining an IL-7 treatment cycle, that is optionally repeated at least once.

8. The IL-7 for use in treating hepatitis C according to any of claims 1 to 6, wherein the patient is to be administered with an antiviral agent or a combination of antiviral agents during a first phase of one week, so as to reduce the viral load, followed by a second phase of four weeks of IL-7 combined with an antiviral agent or a combination of antiviral agents, wherein the antiviral agent or combination of antiviral agents may preferably be the same during all treatment phases.

9. The IL-7 for use in treating hepatitis C according to claim 8, wherein the administration of IL-7 is to be followed by a third phase of 1 to 9 weeks of treatment with an antiviral agent or a combination of antiviral agents, wherein the antiviral agent or combination of antiviral agents may preferably be the same during all treatment phases.

10. The IL-7 for use in treating hepatitis C according to any of claims 1 to 9, wherein the patient has chronic hepatitis C genotype 1 to 6 infection, preferably genotype 1 to 4, preferably genotype 1.

11. The IL-7 for use in treating hepatitis C according to any of claims 1 to 10, for obtaining HCV viral clearance, preventing or delaying onset of liver fibrosis and cirrhosis, and/or preventing relapse of the HCV infection.

12. The IL-7 for use in treating hepatitis C according to any of claims 1 to 11, wherein the IL-7 is in the form of a fusion protein, preferably in fusion with Fc fragment of an immunoglobulin.

13. The IL-7 for use in treating hepatitis C according to any of claims 1 to 12, wherein the IL-7 is a wild-type human IL-7 or a variant thereof, preferably in hyperglycosylated form.

## Patentansprüche

1. Interleukin-7 (IL-7) zur Verwendung in der Behandlung von Hepatitis C in einem Patienten infiziert mit Hepatitis C Virus, wobei der Patient mit einem antiviralen Agens oder einer Kombination von antiviralen Agenzien behandelt worden ist, um die Viruslast zu reduzieren, vor der Verabreichung mit IL-7, wobei das antivirale Agens oder die Kombination von antiviralen Agenzien die Viruslast um weniger als 5 Log₁₀ IU/mL reduziert.

2. IL-7 zur Verwendung in der Behandlung von Hepatitis C nach Anspruch 1, wobei das antivirale Agens oder die Kombination von antiviralen Agenzien die Viruslast um weniger als 4 Log₁₀ IU/ml vor der Verabreichung mit IL-7 reduziert.

3. IL-7 zur Verwendung in der Behandlung von Hepatitis C nach Anspruch 2, wobei das antivirale Agens oder die Kombination von antiviralen Agenzien die Viruslast um weniger als 3 Log₁₀ IU/mL vor Verabreichung mit IL-7 reduziert.

4. IL-7 zur Verwendung in der Behandlung von Hepatitis C nach einem der Ansprüche 1 bis 3, wobei das antivirale Agens ausgewählt ist aus der Gruppe bestehend aus einem Protease-Inhibitor wie Telaprevir oder Boceprevir, einem Polymerase-Inhibitor, einem im Inhibitor des Viruseintritts, und einem Helikase-Inhibitor, oder Kombination davon, optional in Kombination mit Interferon und/oder Ribavirin.

5. IL-7 zur Verwendung in der Behandlung von Hepatitis C nach einem der Ansprüche 1 bis 3, wobei das das antivirale Agens Interferon ist, wie Interferon-alpha oder Consensus-Interferon oder Interferon-lambda, vorzugsweise IFNalpha-2a oder IFNalpha-2b, entweder alleine oder in Kombination mit einem anderen antiviralen Agens, wie Ribavirin, wobei das Interferon vorzugsweise in PEGylierter Form vorliegt.

6. IL-7 zur Verwendung in der Behandlung von Hepatitis C nach einem der Ansprüche 1 bis 5, wobei die Behandlung mit dem antiviralen Agens oder der Kombination von antiviralen Agenzien vor der Behandlung mit IL-7 begonnen wird, und andauert über mindestens einen Teil der Behandlung mit IL-7, vorzugsweise über 6 bis 12 Wochen.

7. IL-7 zur Verwendung in der Behandlung von Hepatitis C nach einem der Ansprüche 1 bis 6, wobei IL-7 einmal pro Woche verabreicht wird, vorzugsweise über eine Dauer von zwei bis sechs Wochen, vorzugsweise vier Wochen, wodurch ein IL-7-Behandlungszyklus definiert wird, der optional mindestens einmal wiederholt wird.

8. IL-7 zur Verwendung in der Behandlung von Hepatitis C nach einem der Ansprüche 1 bis 6, wobei dem Patienten ein antivirales Agens oder eine Kombination für antiviralen Agenzien während einer ersten Phase von einer Woche verabreicht wird, um so die Viruslast zu reduzieren, gefolgt von einer zweiten Phase von vier Wochen von IL-7 kombiniert mit einem antiviralen Agens oder einer Kombination von antiviralen Agenzien, wobei das antivirale Agens oder die Kombination von antiviralen Agenzien vorzugsweise die gleiche während aller Behandlungsphasen sein soll.

9. IL-7 zur Verwendung in der Behandlung von Hepatitis C nach Anspruch 8, wobei die Verabreichung von IL-7 fortgesetzt wird durch eine dritte Phase von 1 bis 9 Wochen der Behandlung mit einem antiviralen Agens oder einer Kombination von antiviralen Agenzien, wobei das antivirale Agens oder die Kombination von antiviralen Agenzien vorzugsweise die gleiche während aller Behandlungsphasen sein soll.

10. IL-7 zur Verwendung in der Behandlung von Hepatitis C nach einem der Ansprüche 1 bis 9, wobei der Patient eine chronische Hepatitis C Genotyp 1 bis 6 Infektion, vorzugsweise Genotyp 1 bis 4, vorzugsweise Genotyp 1, hat.

11. IL-7 zur Verwendung in der Behandlung von Hepatitis C nach einem der Ansprüche 1 bis 10, um HCV virale Beseitigung zu erhalten, Prävention oder Verzögerung des Beginns einer Leberfibrose und -zirrhose und/oder Prävention eines Rückfalls einer HCV Infektion.

12. IL-7 zur Verwendung in der Behandlung von Hepatitis C nach einem der Ansprüche 1 bis 11, wobei IL-7 in der Form eines Fusionsproteins, vorzugsweise in Fusion mit einem Fc-Fragment eines Immunglobulins, vorliegt.

13. IL-7 zur Verwendung in der Behandlung von Hepatitis C nach einem der Ansprüche 1 bis 12, wobei IL-7 ein wild-Typ humanes IL-7 oder eine Variante davon, vorzugsweise in hyperglykosylierter Form, ist.

## Revendications

1. Interleukine-7 (IL-7), pour une utilisation dans le traitement de l'hépatite C chez un patient infecté par le virus de l'hépatite C, dans laquelle le patient a été traité avec un agent antiviral ou une combinaison d'agents antiviraux, de façon à réduire la charge virale, avant administration d'IL-7, dans laquelle l'agent antiviral ou la combinaison d'agents antiviraux réduit la charge virale à moins de 5 Log₁₀ UI/mL.

2. IL-7, pour une utilisation dans le traitement de l'hépatite C selon la revendication 1, dans laquelle l'agent antiviral ou la combinaison d'agents antiviraux réduit la charge virale à moins de 4 Log₁₀ UI/mL avant administration d'IL-7.

3. IL-7, pour une utilisation dans le traitement de l'hépatite C selon la revendication 2, dans laquelle l'agent antiviral ou la combinaison d'agents antiviraux réduit la charge virale à moins de 3 Log₁₀ UI/mL avant administration d'IL-7.

4. IL-7, pour une utilisation dans le traitement de l'hépatite C selon l'une quelconque des revendications 1 à 3, dans laquelle l'agent antiviral est choisi dans le groupe constitué d'un inhibiteur de protéase tel que le Télaprévir ou le Bocéprévir, un inhibiteur de polymérase, un inhibiteur d'entrée du virus, et un inhibiteur d'hélicase, ou une combinaison de ceux-ci, éventuellement en combinaison avec un interféron et/ou la ribavirine.

5. IL-7, pour une utilisation dans le traitement de l'hépatite C selon l'une quelconque des revendications 1 à 3, dans laquelle l'agent antiviral est un interféron, tel que l'interféron alpha ou un interféron consensus ou l'interféron lambda, de préférence l'IFNalpha-2a ou l'IFNalpha-2b, soit seul soit en combinaison avec un autre agent antiviral, tel que la ribavirine, l'interféron étant de préférence sous forme PEGylée.

6. IL-7, pour une utilisation dans le traitement de l'hépatite C selon l'une quelconque des revendications 1 à 5, dans laquelle le traitement avec l'agent antiviral ou la combinaison d'agents antiviraux est commencé avant le traitement avec l'IL-7, et maintenu durant au moins une partie du traitement avec l'IL-7, de préférence durant 6 à 12 semaines.

7. IL-7, pour une utilisation dans le traitement de l'hépatite C selon l'une quelconque des revendications 1 à 6, dans laquelle l'IL-7 est à administrer une fois par semaine, de préférence durant une période de deux à six semaines, de préférence quatre semaines, définissant ainsi un cycle de traitement à l'IL-7, qui est éventuellement répété au moins une fois.

8. IL-7, pour une utilisation dans le traitement de l'hépatite C selon l'une quelconque des revendications 1 à 6, dans laquelle le patient doit être administré avec un agent antiviral ou une combinaison d'agents antiviraux durant une première phase d'une semaine, de façon à réduire la charge virale, suivie d'une seconde phase de quatre semaines d'IL-7 combinée avec un agent antiviral ou une combinaison d'agents antiviraux, dans laquelle l'agent antiviral ou la combinaison d'agents antiviraux peut de préférence être le ou la même durant toutes les phases de traitement.

9. IL-7, pour une utilisation dans le traitement de l'hépatite C selon la revendication 8, dans laquelle l'administration d'IL-7 doit être suivie d'une troisième phase de 1 à 9 semaines de traitement avec un agent antiviral ou une combinaison d'agents antiviraux, dans laquelle l'agent antiviral ou la combinaison d'agents antiviraux peut de préférence être le ou la même durant toutes les phases de traitement.

10. IL-7, pour une utilisation dans le traitement de l'hépatite C selon l'une quelconque des revendications 1 à 9, dans laquelle le patient a une hépatite C chronique de génotype 1 à 6, de préférence de génotype 1 à 4, de préférence de génotype 1.

11. IL-7, pour une utilisation dans le traitement de l'hépatite C selon l'une quelconque des revendications 1 à 10, pour obtenir une clairance virale du HCV, prévenir ou retarder l'apparition de fibrose et cirrhose hépatiques, et/ou prévenir la récidive d'une infection au HCV.

12. IL-7, pour une utilisation dans le traitement de l'hépatite C selon l'une quelconque des revendications 1 à 11, dans laquelle l'IL-7 est sous la forme d'une protéine de fusion, de préférence en fusion avec le fragment Fc d'une immunoglobuline.

13. IL-7, pour une utilisation dans le traitement de l'hépatite C selon l'une quelconque des revendications 1 à 12, dans laquelle l'IL-7 est une IL-7 humaine sauvage ou un variant de celle-ci, de préférence sous forme hyperglycosylée.


### SEQUENCE LISTING

<110> Cytheris
<120> HCV immunotherapy
<130> B1220
<160> 1
<170> PatentIn version 3.1
<210> 1
   <211> 152
   <212> PRT
   <213> Homo sapiens
<400> 1
   Asp Cys Asp Ile Glu Gly Lys Asp Gly Lys Gln Tyr Glu Ser Val Leu
   1               5                   10                  15
   Met Val Ser Ile Asp Gln Leu Leu Asp Ser Met Lys Glu Ile Gly Ser
               20                  25                  30
   Asn Cys Leu Asn Asn Glu Phe Asn Phe Phe Lys Arg His Ile Cys Asp
           35                  40                  45
   Ala Asn Lys Glu Gly Met Phe Leu Phe Arg Ala Ala Arg Lys Leu Arg
       50                  55                  60
   Gln Phe Leu Lys Met Asn Ser Thr Gly Asp Phe Asp Leu His Leu Leu
   65                  70                  75                  80
   Lys Val Ser Glu Gly Thr Thr Ile Leu Leu Asn Cys Thr Gly Gln Val
                   85                  90                  95
   Lys Gly Arg Lys Pro Ala Ala Leu Gly Glu Ala Gln Pro Thr Lys Ser
               100                 105                 110
   Leu Glu Glu Asn Lys Ser Leu Lys Glu Gln Lys Lys Leu Asn Asp Leu
           115                 120                 125
   Cys Phe Leu Lys Arg Leu Leu Gln Glu Ile Lys Thr Cys Trp Asn Lys
       130                 135                 140
   Ile Leu Met Gly Thr Lys Glu His
   145                 150